# EUROPEAN PATENT SPECIFICATION

(11) **EP 4 467 115 B1**
(45) Date of publication and mention of the grant of the patent: **14.10.2026**
(21) Application number: 23746435.9
(22) Date of filing: 29.01.2023
(51) Int. Cl.: A61F 13/15, A61F 13/42

(54) **ABSORPTION PRODUCT AND MANUFACTURING METHOD THEREFOR, AND PRODUCTION SYSTEM FOR MANUFACTURING ABSORPTION PRODUCT**
ABSORPTIONSPRODUKT UND HERSTELLUNGSVERFAHREN DAFÜR SOWIE HERSTELLUNGSSYSTEM ZUR HERSTELLUNG DES ABSORPTIONSPRODUKTS
PRODUIT D'ABSORPTION ET SON PROCÉDÉ DE FABRICATION, ET SYSTÈME DE PRODUCTION POUR LA FABRICATION D'UN PRODUIT D'ABSORPTION

(30) Priority: 29.01.2022 CN 202210111332
(43) Date of publication of application: 27.11.2024
(73) Proprietor: Shenda Chuangxin (Shenzhen) Technology Co., Ltd, Shenzhen, Guangdong 518051 (CN)
(72) Inventor: XIE, Jiquan, Shenzhen, Guangdong 518051 (CN); XIE, Junda, Shenzhen, Guangdong 518051 (CN)
(74) Representative: Reddie & Grose LLP
(86) International application number: PCT/CN2023/073674
(87) International publication number: WO 2023/143558

(56) References cited:
- CN-A- 112 535 578
- CN-A- 114 469 526
- CN-U- 203 777 158
- US-A1- 2011 319 845
- US-A1- 2019 167 489
- US-A1- 2019 167 490

## Description

### TECHNICAL FIELD

The present application relates to the technical field of absorption products, in particular to an absorption product, a manufacturing method therefor, and a production system for manufacturing an absorption product.

### BACKGROUND

Absorption products include diapers, sanitary napkins, incontinence products, etc. The diaper generally includes a surface covering layer, an absorbent core layer, and a base fabric. The surface covering layer is close to the baby's body, which can promote the rapid penetration of urine and effectively prevent the back-seepage, and keep the surface of the diaper dry.

With the improvement of living standards, people's demand for disposable absorption products is no longer just satisfied with the function of absorbing urine, and the people's demand begins to become multi-functional. Therefore, so a variety of smart diapers have emerged in the market.

However, in the conventional production process of the smart diaper, after a main body of the smart diaper is manufactured, a mounting position of a data reader needs to be set according to a position of a sensing layer of the main body of the smart diaper, so that the production efficiency of the smart diaper is low.

US 2019/167489 discloses a substrate suitable for incorporation into an absorbent article for automatic detection of wetness events therein, the substrate comprising a first surface capable of being arranged proximal to a body facing side of the absorbent article and a second surface opposite said first surface and capable of being arranged proximal to a garment facing side of said absorbent article, said substrate comprising a plurality of sensor tracks disposed on said first surface and said sensor tracks comprising: at least one central track extending parallel to a length of the substrate and parallel to a longitudinal axis crossing a first end and a second end of the substrate; at least two side tracks extending parallel to the central track and oppositely arranged such that the central track extends therebetween; and wetness sensing tracks extending outboard of said two side tracks, wherein said central track, said side tracks, and said wetness sensing tracks are in electrical communication via one or more shortening elements positioned proximal to said second end and distal from said first end, and wherein the substrate is connectable to a clip-on data processing module at a position proximal to said first end and distal from said shortening elements such to form a closed electrical circuit, typically for measuring resistance and/or capacitance therethrough.

### SUMMARY

Therefore, a technical problem to be solved by the present application is to provide an absorption product, a manufacturing method therefor, and a production system for manufacturing the absorption product, which can improve the production efficiency of smart diapers.

In order to solve the above problem, the present application provides a manufacturing method for an absorbent product, including:
supplying and conveying a surface layer and a leakage-proof bottom layer along a longitudinal direction, wherein the surface layer and the leakage-proof bottom layer are opposite to each other and spaced apart, and an absorbent core having a preset size is provided between the surface layer and the leakage-proof bottom layer;
supplying and conveying a sensing layer, the sensing layer being located on a side of the surface layer;
acquiring an in-position signal of a cutting mark on the leakage-proof bottom layer, and controlling a cutting of the sensing layer according to the in-position signal of the cutting mark to obtain a sensing strip having a preset length;
adhering the sensing strip obtained by cutting to a side of the surface layer facing the absorbent core;
controlling a cutting of the leakage-proof bottom layer according to the in-position signal of the cutting mark, so as to form a receiving groove at a preset position of the leakage-proof bottom layer, wherein the receiving groove is configured to receive a data reader, and an orthographic projection of the sensing strip on the leakage-proof bottom layer intersects with the receiving groove, and the data reader is configured to acquire a conduction data of the sensing strip; and
adhering the surface layer, the absorbent core, and the leakage-proof bottom layer sequentially, so that one end of the sensing strip is capable of being connected to the data reader in the receiving groove, and another end of the sensing strip is capable of being in contact with the absorbent core.

Optionally, controlling the cutting of the sensing layer according to the in-position signal of the cutting mark to obtain the sensing strip having the preset length specifically includes:
setting a duration of continuous cutting of the sensing layer according to the in-position signal of the cutting mark;
wherein the duration of the continuous cutting of the sensing layer is positively correlated with the preset length.

Optionally, an interval time between a start time of cutting the sensing layer and a start time of cutting the leakage-proof bottom layer is set according to a position of the sensing strip relative to the receiving groove in the longitudinal direction.

Optionally, the sensing layer is cut at a preset speed according to a conveying speed of the sensing layer.

Optionally, a plurality of cutting marks are spaced apart on the leakage-proof bottom layer along the longitudinal direction.

Optionally, adhering the sensing strip obtained by cutting to the side of the surface layer facing the absorbent core includes:
adsorbing the sensing strip obtained by cutting to a first cutting roller configured to cut the sensing strip;
controlling the first cutting roller to rotate, so as to attach the adsorbed sensing strip to the side of the surface layer facing the absorbent core

Optionally, controlling the cutting of the leakage-proof bottom layer according to the in-position signal of the cutting mark specifically includes:
cutting the leakage-proof bottom layer, and sucking a waste material obtained by cutting into a hollow cavity of a second cutting roller configured to cut the leakage-proof bottom layer, so that the waste material obtained by cutting is capable of being discharged through the hollow cavity of the second cutting roller.

Optionally, a process of manufacturing the absorbent core includes:
supplying and conveying an absorbent core layer in the longitudinal direction;
controlling a cutting of the absorbent core layer according to the in-position signal of the cutting mark to obtain the absorbent core having the preset size; and
adhering the absorbent core to a side of the leakage-proof bottom layer facing the surface layer, so that an orthographic projection of the absorbent core on the leakage-proof bottom layer and the receiving groove are staggered with each other.

The present application provides a production system for manufacturing an absorption product, including a controller storing a computer program that, when executed by the controller, causes the controller to perform the steps of the aforementioned manufacturing method.

The present application provides an absorption product, including:
a surface layer arranged along a longitudinal direction;
a sensing strip having a preset length along the longitudinal direction, the sensing strip being adhered to a side of the surface layer;
a leakage-proof bottom layer provided on a side of the surface layer adhered to the sensing strip, wherein a receiving groove configured to receive a data reader is formed at a preset position of the leakage-proof bottom layer, and an orthographic projection of the sensing strip on the leakage-proof bottom layer intersects with the receiving groove; and
an absorbent core provided between the surface layer and the leakage-proof bottom layer;
wherein an end of the sensing strip is capable of being connected to the data reader in the receiving groove, and another end of the sensing strip is capable of being in contact with the absorbent core.

The present application provides the absorption product and the manufacturing method thereof, and the production system for manufacturing an absorption product. In the manufacturing method of the absorption product, the cutting of the sensing layer and the cutting of the leakage-proof bottom layer are controlled according to the in-position signal of the same cutting mark, so that the orthographic projection of the sensing strip on the leakage-proof bottom layer intersects with the receiving groove configured to receive the data reader. Thus, one end of the obtained sensing strip having the preset length can be connected to the data reader in the receiving groove, and the other end of the obtained sensing strip is can be in contact with the absorbent core. When the absorption product is in use, the absorbent liquid, such as urine, can pass through the surface layer and penetrate into the absorbent core. In this process, the end of the sensing strip in contact with the absorbent core is conducted through the part of the absorbent liquid, and the data reader can acquire a conduction data of the sensing strip to send out a replacement prompt, so that the absorption product can be replaced in time according to the replacement prompt. It should be understood that, in the manufacturing method for the absorption product, a mounting position of the data reader is not required to be additionally set according to the position of the sensing strip, which can effectively improve the production efficiency.

### BRIEF DESCRIPTION OF THE DRAWINGS

To illustrate the technical solutions according to the embodiments of the present application or in the prior art more clearly, the accompanying drawings for describing the embodiments or the prior art are introduced briefly in the following. Apparently, the accompanying drawings in the following description are only some embodiments of the present application, and persons of ordinary skill in the art can derive other drawings from the accompanying drawings without creative efforts.
FIG. 1 is a flowchart of a manufacturing method for an absorption product according to an embodiment of the present application.
FIG. 2 is a schematic diagram of a process of adhering a sensing strip to a side of a surface layer according to an embodiment of the present application.
FIG. 3 is a schematic diagram of a process of forming a receiving groove on a leakage-proof bottom layer according to an embodiment of the present application.
FIG. 4 is a schematic structural diagram of the surface layer and the sensing strip according to an embodiment of the present application.
FIG. 5 is a schematic structural diagram of the leakage-proof bottom layer according to an embodiment of the present application.
FIG. 6 is a schematic structural diagram of an absorption product according to an embodiment of the present application.
FIG. 7 is a schematic structural diagram of the sensing strip according to an embodiment of the present application.
FIG. 8 is a schematic diagram of a cutting principle of the leakage-proof bottom layer according to an embodiment of the present application.

Reference numerals are indicated as:
20. Absorption product; 210. Surface layer; 211. Surface main body layer; 212. Elastic edge separation layer; 220. Leakage-proof bottom layer; 221. Receiving groove; 230. Absorbent core; 240. Sensing layer; 241. Sensing strip; 2411. Sensing sub-strip; 250. Cutting mark; 260. Data reader; 271. First cutting roller; 2711. Fixed inner roller; a. Adsorption cavity; 2712. Rotating outer roller; b. Adsorption hole; 2713. Arc-shaped cutter; 272. Second cutting roll; 2721. Hollow cavity.

### DETAILED DESCRIPTION OF THE EMBODIMENTS

In order to make the above objects, features and advantages of the present application clear and easier to understand, the specific embodiments of the present application are described in detail below in combination with the accompanying drawings. Many specific details are set forth in the following description to facilitate a full understanding of the present application. However, the present application can be implemented in many ways different from those described herein, and those skilled in the art can make similar improvements without departing from the connotation of the present application. Therefore, the present application is not limited by the specific embodiments disclosed below.

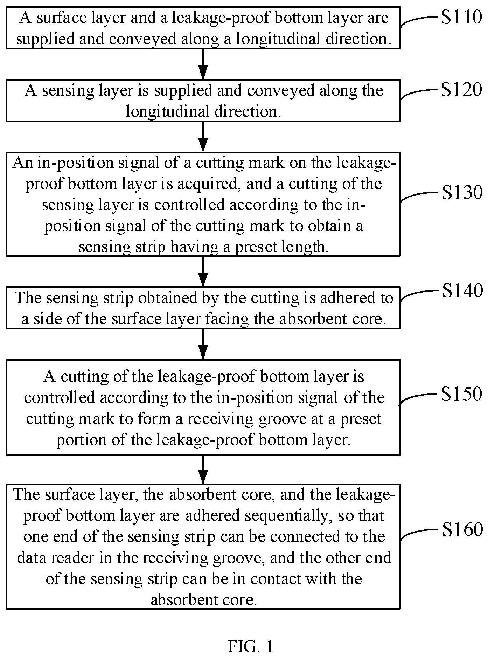

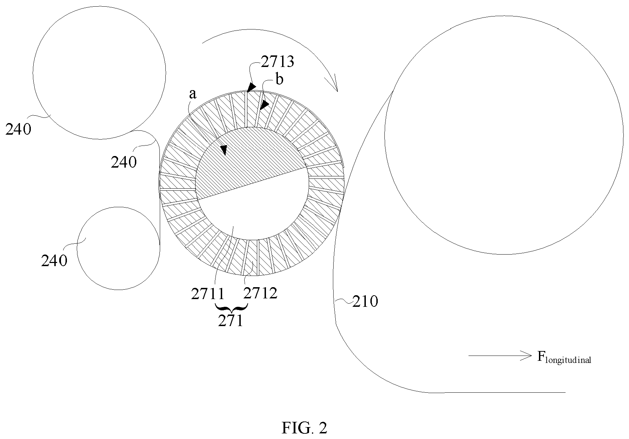

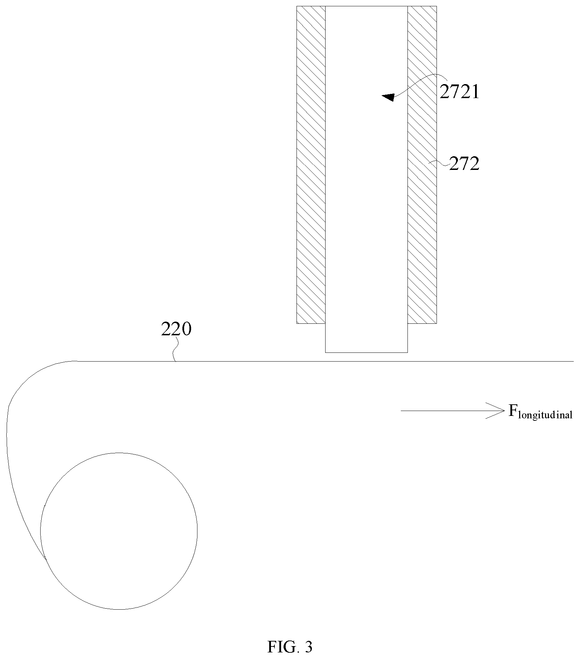

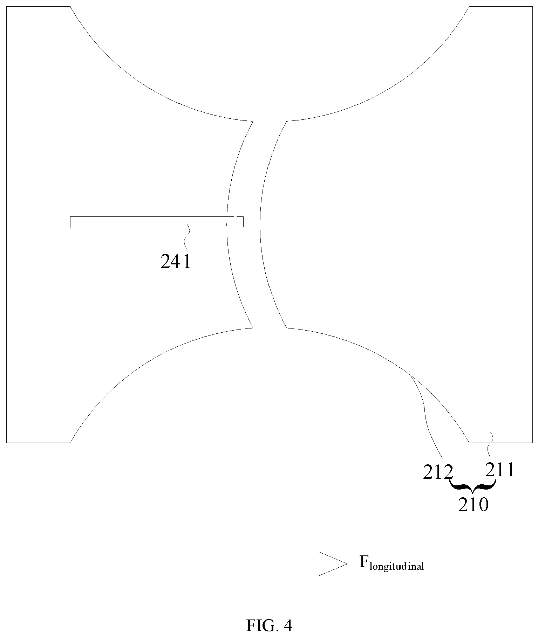

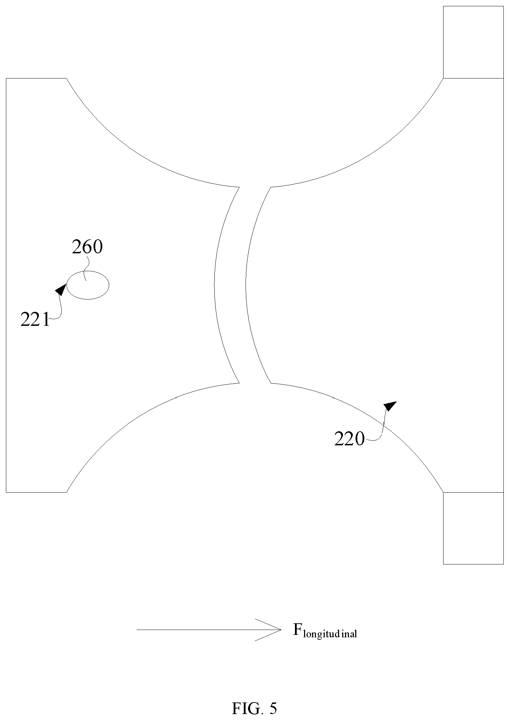

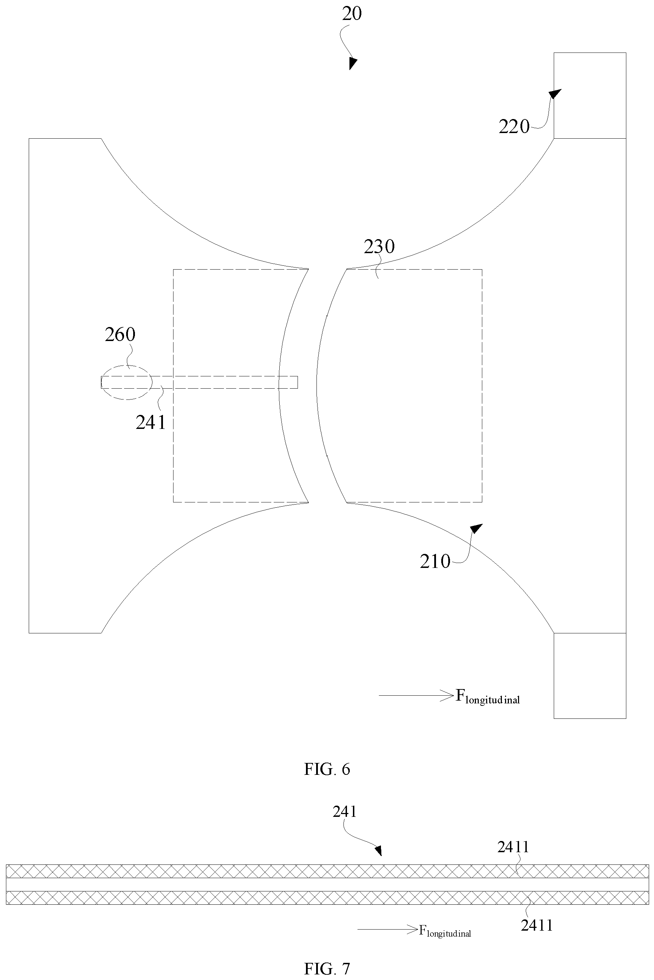

Referring to FIG. 1, in conjunction with FIG. 2 and FIG. 3, according to an embodiment of the present application, the present application provides a manufacturing method for an absorption product 20, including the following steps:
S110. A surface layer 210 and a leakage-proof bottom layer 220 are supplied and conveyed along a longitudinal direction F_{longitudinal}. The surface layer 210 and the leakage-proof bottom layer bottom layer 220 are opposite to each other and spaced apart, and an absorbent core 230 having a preset size is provided between the surface layer 210 and the leakage-proof bottom layer 220. The absorbent core 230 may be laid on a side of the leakage-proof bottom layer 220 facing the surface layer 210.
S120. A sensing layer 240 is supplied and conveyed, and the sensing layer 240 is located on a side of the surface layer 210.
S130. An in-position signal of a cutting mark 250 on the leakage-proof bottom layer 220 is acquired, and a cutting of the sensing layer 240 is controlled according to the in-position signal of the cutting mark 250 to obtain a sensing strip 241 having a preset length.
S140. The sensing strip 241 obtained by cutting is adhered to a side of the surface layer 210 facing the absorbent core 230 (as shown in FIG.4).
S150. A cutting of the leakage-proof bottom layer 220 is controlled according to the in-position signal of the cutting mark 250 to form a receiving groove 221 (as shown in FIG. 5) at a preset portion of the leakage-proof bottom layer 220. The receiving groove 221 is configured to receive a data reader 260, and an orthographic projection of the sensing strip 241 on the leakage-proof bottom layer 220 intersects with the receiving groove 221 (as shown in FIG. 6). In this way, an end of the obtained sensing strip 241 can be electrically connected to the data reader 260 in the receiving groove 221, so that the data reader 260 can obtain a data related to an absorbent liquid on the absorbent core 230 through the sensing strip 241.

It should be noted that the preset portion of the leakage-proof bottom layer 220 is located on the side of the leakage-proof bottom layer 220 facing the surface layer 210.

S160. The surface layer 210, the absorbent core 230, and the leakage-proof bottom layer 220 are adhered sequentially, so that one end of the sensing strip 241 can be connected to the data reader 260 in the receiving groove 221, and the other end of the sensing strip 241 can be in contact with the absorbent core 230.

In the manufacturing method of the absorption product 20, the cutting of the sensing layer 240 and the cutting of the leakage-proof bottom layer 220 are controlled according to the in-position signal of the same cutting mark 250, so that the orthographic projection of the sensing strip 241 on the leakage-proof bottom layer 220 intersects with the receiving groove 221 configured to receive the data reader 260. Thus, one end of the obtained sensing strip 241 having the preset length can be connected to the data reader 260 in the receiving groove 221, and the other end of the obtained sensing strip 241 is can be in contact with the absorbent core 230. When the absorption product 20 is in use, the absorbent liquid, such as urine, can pass through the surface layer 210 and penetrate into the absorbent core 230. In this process, the end of the sensing strip 241 in contact with the absorbent core 230 is conducted through the part of the absorbent liquid, and the data reader 260 can acquire a conduction data of the sensing strip 241 to send out a replacement prompt, so that the absorption product 20 can be replaced in time according to the replacement prompt. It should be understood that, in the manufacturing method for the absorption product 20, a mounting position of the data reader 260 is not required to be additionally set according to a position of the sensing strip 241, which can effectively improve the production efficiency.

It should be noted that, referring to FIG. 7, the sensing strip 241 includes two sensing sub-strips 2411 spaced apart in a direction perpendicular to the longitudinal direction F_{longitudinal} and parallel to the sensing strip 241. Before the absorption product 20 absorbs urine, ends of the two sensing strips 2411 away from the data reader 260 are disconnected from each other.

Referring to FIG. 6, in the longitudinal direction F_{longitudinal}, the orthographic projection of the sensing strip 241 on the leakage-proof bottom layer 220 intersects with the receiving groove 221.

In an embodiment, the cutting of the sensing layer 240 is controlled according to the in-position signal of the cutting mark 250 to obtain the sensing strip 241 having the preset length, which specifically includes:

A duration of the continuous cutting of the sensing layer 240 is set according to the in-position signal of the cutting mark 250. The duration of the continuous cutting of the sensing layer 240 is positively correlated with the preset length. It should be understood that the sensing layer 240 is continuously cut, and the duration of the continuous cutting is positively correlated with the preset length, so that the sensing strip 241 having the preset length can be obtained.

Optionally, the sensing layer 240 may be cut by an arc-shaped cutter 2713. The arc-shaped cutter 2713 includes an arc-shaped main body portion and an annular cutter provided on the arc-shaped main body portion and extending along a bending direction of the arc-shaped main body portion. The arc-shaped cutter 2713 is controlled to rotate to continuously cut the sensing layer 240, and the sensing strip 241 having the preset length can be obtained.

In one embodiment, the sensing layer 240 is cut at a preset speed according to a conveying speed of the sensing layer 240. In this way, a cutting accuracy of the cut sensing strip 241 can be better ensured.

Optionally, a rotation speed of the arc-shaped cutter 2713 is set according to the conveying speed of the sensing layer 240, so as to improve the cutting accuracy of the sensing strip 241.

In an embodiment, an interval time between a start time of cutting the sensing layer 240 and a start time of cutting the leakage-proof bottom layer 220 is set according to a position of the sensing strip 241 relative to the receiving groove 221 in the longitudinal direction F_{longitudinal}. The interval time between the start time of cutting the sensing layer 240 and the start time of cutting the leakage-proof bottom layer 220 may be set according to a relative positional relationship between the orthographic projection of the sensing strip 241 on the leakage-proof bottom layer 220 and the receiving groove 221 in the longitudinal direction F_{longitudinal} in the absorption product 20. In this way, it can be ensured that in the finally manufactured absorption product 20, the orthographic projection of the sensing strip 241 on the leakage-proof bottom layer 220 in the longitudinal direction F_{longitudinal} intersects with the receiving groove 221, and that one end of the sensing strip 241 can be electrically connected to the data reader 260 received in the receiving groove 221, thereby improving the reliability of data reading of the absorption product 20.

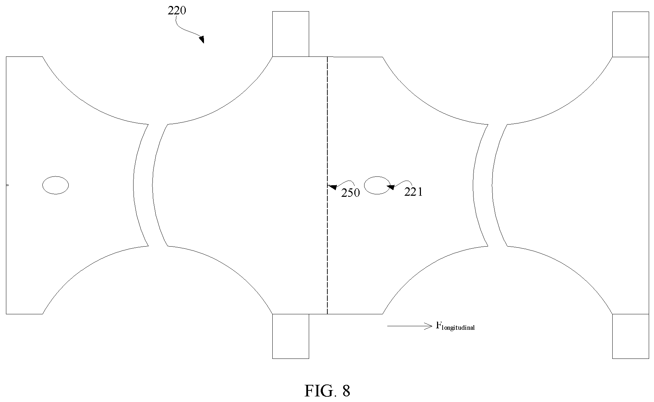

In an embodiment, referring to FIG. 8, a plurality of cutting marks 250 are spaced apart on the leakage-proof bottom layer 220 along the longitudinal direction F_{longitudinal}.

The cutting of the sensing layer 240 and the leakage-proof bottom layer 220 is controlled according to the in-position signal of the cutting mark 250. In this way, the sensing layer 240 and the leakage-proof bottom layer 220 are cut respectively with the in-position signal of any one of the cutting marks 250 as a reference, so as to ensure that in the finally manufactured absorption product 20, the orthographic projection of the sensing strip 241 on the leakage-proof bottom layer 220 in the longitudinal direction F_{longitudinal} can intersect with the receiving groove 221, thereby ensuring that one end of the sensing strip 241 can be well electrically connected to the data reader 260 received in the receiving groove 221. In addition, the plurality of cutting marks 250 are spaced apart on the bottom leakage-proof bottom layer 220 along the longitudinal direction F_{longitudinal}. The plurality of cutting marks 250 can be used to respectively cut the sensing layer 240 and the leakage-proof bottom layer 220 for a plurality of times, so as to facilitate the manufacturing of the plurality of absorption products 20.

In an embodiment, referring to FIG. 2, the sensing strip 241 obtained by cutting is adhered to the side of the surface layer 210 facing the absorbent core 230, which specifically includes:

The sensing strip 241 obtained by cutting is adsorbed to a first cutting roller 271 configured to cut the sensing strip 241.

The first cutting roller 271 is controlled to rotate to attach the adsorbed sensing strip 241 to the side of the surface layer 210 facing the absorbent core 230.

In this way, after the first cutting roller 271 of the first cutting roller 271 is controlled to cut the sensing layer 240, the sensing strip 241 obtained by cutting can be transferred to the side of the surface layer 210 facing the absorbent core 230 by the first cutting roller 271, and the adsorption of the sensing strip 241 can be released, so that the sensing strip 241 can be transferred to the side of the surface layer 210 facing the absorbent core 230, and the production efficiency of the absorption product 20 can be further improved.

Optionally, the first cutting roller 271 includes a fixed inner roller 2711 and a rotating outer roller 2712 rotatably connected to the fixed inner roller 2711 and surrounding the fixed inner roller 2711. The fixed inner roller 2711 is provided with an adsorption cavity an externally connected to a suction member. The rotating outer roller 2712 is provided with a plurality of adsorption suction holes b uniformly distributed on an outer peripheral surface of the rotating outer roller 2712. The adsorption cavity a is in communication with a part of the adsorption holes b. The first cutting roller 271 further includes the arc-shaped cutter 2713 provided on the rotating outer roller 2712 and located outside the adsorption cavity a, and the arc-shaped cutter 2713 surrounds a part of the adsorption holes b. In this way, during the rotation of the rotating outer roller 2712, the sensing layer 240 can be cut to obtain the sensing strip 241 of the preset length, and the cut sensing strip 241 can be adsorbed by the adsorption holes b on the first cutting roller 271 and rotate with the rotating outer roller 2712. When one end of the sensing strip 241 rotates to face the surface layer 210, the adsorption holes b on a position of the rotating outer roller 2712 attached to the surface layer 210 are not in communication with the adsorption cavity a, and the sensing strip 241 is gradually separated from the adsorption, so that the sensing strip 241 is adhered to one side of the surface layer 210.

Optionally, a rotation speed of the rotating outer roller 2712 is set according to the conveying speed of the sensing layer 240. Thus, the rotation speed of the arc-shaped cutter 2713 is set, and the cutting accuracy of the sensing strip 241 can be improved.

In some embodiments, the first cutting roller 271 is controlled to rotate to attach the adsorbed sensing strip 241 to the side of the surface layer 210 facing the absorbent core 230, which specifically includes:

A hot melt adhesive spraying machine is controlled to spray adhesive on a side of the surface layer 210, so that the sensing strip 241 can be adhered to the side of the surface layer 210 facing the absorbent core 230. It can be ensured that the sensing strip 241 is adhered to the side of the surface layer 210 facing the absorbent core 230.

Optionally, the number of adhesive heads of the hot melt adhesive spraying machine is selected according to a width size of the sensing strip 241, so that all the adhesive heads of the hot melt adhesive spraying machine can be spaced apart along a width direction of the sensing strip 241. The width size of the sensing strip 241 is a size of the sensing strip 241 perpendicular to the longitudinal direction. In this way, the sensing strip 241 can be firmly adhered to one side of the surface layer 210.

Optionally, a size of an adhesive droplet sprayed from the adhesive head of the hot melt adhesive spraying machine is controlled to be between 1 mm and 8 mm, preferably between 1 mm and 2 mm. A time interval between two adjacent adhesive droplets is set, so as to apply adhesive to one side of the surface layer 210.

In some embodiments, referring to FIG. 3, the step 150 of controlling the cutting of the leakage-proof bottom layer 220 according to the in-position signal of the cutting mark 250 specifically includes:

The leakage-proof bottom layer 220 is cut, and a waste material obtained by cutting is sucked into a hollow cavity 2721 of a second cutting roller 272 configured to cut the leakage-proof bottom layer 220, so that the waste material obtained by cutting can be discharged through the hollow cavity 2721 of the second cutting roller 272. In this way, the waste material obtained by cutting can be discharged while the leakage-proof bottom layer 220 is cut, thereby improving the production efficiency of the absorption product 20.

Optionally, the second cutting roller 272 may have a suitable shape according to a shape of the data reader 260. Specifically, a blade of the second cutting roller 272 is elliptical, and a size of the blade of the second cutting roller 272 is 30 mm * 20 mm. In this way, a size of a notch of the receiving groove 221 formed on the leakage-proof bottom layer 220 is also 30 mm * 20 mm, which facilitates the mounting of the data reader 260.

Optionally, a straight line passing through one side of the cutting mark 250 in the longitudinal direction F_{longitudinal} may be a reference line. Specifically, in the embodiment shown in FIG. 8, the reference line is a dotted line as shown in the figure. According to a preset interval between the receiving groove 221 and the reference line in the longitudinal direction F_{longitudinal}, a position of the second cutting roller 272 relative to the reference line is adjusted, and the second cutting roller 272 is controlled to cut the leakage-proof bottom layer 220. In this way, an interval between the receiving groove 221 formed on the leakage-proof bottom layer 220 and the reference line along the longitudinal direction F_{longitudinal} is approximately equal to the preset interval, which satisfies the setting requirement.

In an embodiment, referring to FIG. 4, the surface layer 210 includes a surface main body layer 211 and two elastic edge separation layers 212 attached to two opposite sides of the surface main body layer 211. The manufacturing process of the surface layer 210 is as follows:

The surface main body layer 211 is wound on a first air expansion reel, so that an end of the surface main body layer 211 away from the first air expansion reel passes through a first buffer unwinder, a deviation corrector, and an adhesive spraying head, and is supplied and conveyed along the longitudinal direction F_{longitudinal}.

A leakage-proof edge separation material is wound on a second air expansion reel, so that an end of the leakage-proof separation edge material away from the second air expansion reel passes through a second buffer unwinder and is supplied and conveyed along the longitudinal direction F_{longitudinal}.

The leakage-proof edge separation material passing through the second buffer unwinder is cut in half, two elastic spandex yarns are continuously laid on the cut leakage-proof edge separation material, respectively, and the leakage-proof edge separation material laid with the elastic spandex yarns is folded using a folding plate to form the elastic edge separation layer 212.

The obtained two elastic edge separation layers 212 are respectively attached to two opposite sides of the surface main body layer 211 to obtain the surface layer 210.

In this way, if the absorption product 20 is a diaper, the absorption product 20 manufactured by using the surface layer 210 can be well attached to a user and has a good leakproof effect.

In an embodiment, the manufacturing process of the leakage-proof bottom layer 220 is as follows:
A bottom non-woven fabric material and a bottom air permeable film material are adhered to obtain a bottom substrate layer.

A Velcro material is attached to a waist patch non-woven fabric, and the attached whole is divided into two halves to obtain a left waist patch layer and a right waist patch layer, respectively.

The left waist patch layer and the right waist patch layer are attached to two opposite sides of the bottom substrate layer, respectively.

A non-woven fabric front waist patch material after passing through a buffer, the deviation corrector, and a positioner is cut into a strip shape and attached to the bottom non-woven fabric material to obtain the leakage-proof bottom layer 220.

In some embodiments, a process of manufacturing the absorbent core 230 includes:

The absorbent core lay is supplied and conveyed in the longitudinal direction F_{longitudinal}.

The cutting of an absorbent core layer is controlled according to the in-position signal of the cutting mark 250 to obtain the absorbent core 230 having the preset size.

The absorbent core 230 is adhered to the side of the leakage-proof bottom layer 220 facing the surface layer 210, so that an orthographic projection of the absorbent core 230 on the leakage-proof bottom layer 220 and the receiving groove 221 are staggered from each other. Specifically, in the longitudinal direction F_{longitudinal}, the orthographic projection of the absorbent core 230 on the leakage-proof bottom layer 220 and the receiving groove 221 are staggered from each other.

In this way, the absorbent core 230 can be centered and adhered to the side of the leakage-proof bottom layer 220 facing the surface layer 210, and the data reader 260 mounted in the receiving groove 221 can acquire the signal data of the absorbent liquid absorbed by the absorbent core 230 through the sensing strip 241.

Optionally, one end of the sensing strip 241 can be connected to the data reader 260 in the receiving groove 221, and the other end of the sensing strip 241 can be in contact with a middle portion of the absorbent core 230. In this way, when the manufactured absorption product 20 is used, the absorbent liquid such as urine can penetrate into a middle area of the absorbent core 230 through the surface layer 210. In this process, the end of the sensing strip 241 in contact with the absorbent core 230 is conducted through this part of the absorbent liquid, and the data reader 260 can obtain the conduction data of the sensing strip 241 to send out the replacement prompt, so as to inform the user to replace the absorption product 20. The data reader 260 can respond more quickly when the absorption product 20 absorbs a certain amount of absorbent liquid to send out the replacement prompt more quickly.

In some embodiments, after the surface layer 210, the absorbent core 230, and the leakage-proof bottom layer 220 are sequentially adhered, the manufacturing method for the absorption product 20 further includes cutting the adhered whole to obtain a plurality of absorption products 20.

The present application provides a production system for manufacturing an absorption product 20, including a controller storing a computer program, and the steps of the manufacturing method are realized when the computer program is executed by the controller.

Optionally, an air permeable film of the leakage-proof bottom layer 220 may be provided with a printed pattern, the cutting mark 250 is configured as a positioning cursor on a side edge of the printed pattern along the longitudinal direction F_{longitudinal}. The production system for manufacturing absorption product 20 further include a photoelectric sensor located above the leakage-proof bottom layer 220. The photoelectric sensor is electrically connected to the controller and is configured to acquire the in-position signal of the cutting mark 250 on the leakage-proof bottom layer 220. The controller cuts the leakage-proof bottom layer 220 and the sensing layer 240 according to the in-position signal of the cutting mark 250, so that in the manufactured absorption product 20, one end of the sensing strip 241 can be connected to the data reader 260 in the receiving groove 221, and the other end of the sensing strip 241 can be in contact with the absorbent core 230.

Optionally, the positioning cursor may be a black cursor, a blue cursor, a dark brown cursor, etc., preferably a black cursor. A size of the positioning cursor is c*d, where c is 5 mm to 8 mm, d is 2 mm to 5 mm, and preferably the black cursor is 5 mm*2 mm.

The present application provides the absorption product 20, which includes the surface layer 210, the sensing strip 241, and the leakage-proof bottom layer 220. The surface layer 210 is provided along the longitudinal direction F_{longitudinal}. The sensing strip 241 has the preset length along the longitudinal direction F_{longitudinal}. The sensing strip 241 is adhered to a side of the surface layer 210.

The leakage-proof bottom layer 220 is provided on a side of the surface layer 210 adhered to the sensing strip 241. The receiving groove 221 configured to receive the data reader 260 is formed at a preset position of the leakage-proof bottom layer 220, and the orthographic projection of the sensing strip 241 on the leakage-proof bottom layer 220 intersects with the receiving groove 221. In this way, one end of the sensing strip 241 can be electrically connected to the data reader 260 in the receiving groove 221.

The absorbent core 230 is provided between the surface layer 210 and the leakage-proof bottom layer 220. One end of the sensing strip 241 can be connected to the data reader 260 in the receiving groove 221, and the other end of the sensing strip 241 can be in contact with the absorbent core 230. In this way, the sensing strip 241 connected to the data reader 260 is conducted through the absorbent liquid absorbed by the absorption product 20, so that a replacement prompt is sent out when the absorption product 20 absorbs a certain amount of absorbent liquid.

In some embodiments, the sensing layer 240 includes two base material layers and an electrically conductive layer provided on the two base material layers, and the electrically conductive layer includes two electrically conductive strips spaced apart. The base material layer is made of soft PET/PP or PET/PP. The PET material after special treatment has good flexibility, and has the capability of being wound into a loop with a length of at least 50mm.

Optionally, the sensing layer 240 may be wound into a coil with a diameter of 276 mm.

Optionally, the sensing layer 240 has a length of 500 m to 1600 m, preferably 800 m, in the longitudinal direction F_{longitudinal}.

## Claims

1. A manufacturing method for an absorption product (20), comprising:
supplying and conveying a surface layer (210) and a leakage-proof bottom layer (220) along a longitudinal direction, wherein the surface layer (210) and the leakage-proof bottom layer (220) are opposite to each other and spaced apart, and an absorbent core (230) having a preset size is provided between the surface layer (210) and the leakage-proof bottom layer (220);
supplying and conveying a sensing layer (240), the sensing layer (240) being located on a side of the surface layer (210);
acquiring an in-position signal of a cutting mark (250) on the leakage-proof bottom layer (220), and controlling a cutting of the sensing layer (240) according to the in-position signal of the cutting mark (250) to obtain a sensing strip (241) having a preset length;
adhering the sensing strip (241) obtained by cutting to a side of the surface layer (210) facing the absorbent core (230);
controlling a cutting of the leakage-proof bottom layer (220) according to the in-position signal of the cutting mark (250), so as to form a receiving groove (221) at a preset position of the leakage-proof bottom layer (220), wherein the receiving groove (221) is configured to receive a data reader (260), and an orthographic projection of the sensing strip (241) on the leakage-proof bottom layer (220) intersects with the receiving groove (221), and the data reader (260) is configured to acquire a conduction data of the sensing strip (241); and
adhering the surface layer (210), the absorbent core (230), and the leakage-proof bottom layer (220) sequentially, so that one end of the sensing strip (241) is capable of being connected to the data reader (260) in the receiving groove (221), and another end of the sensing strip (241) is capable of being in contact with the absorbent core (230).

2. The manufacturing method for the absorption product (20) according to claim 1, wherein controlling the cutting of the sensing layer (240) according to the in-position signal of the cutting mark (250) to obtain the sensing strip (241) having the preset length specifically comprises:
setting a duration of continuous cutting of the sensing layer (240) according to the in-position signal of the cutting mark (250);
wherein the duration of the continuous cutting of the sensing layer (240) is positively correlated with the preset length.

3. The manufacturing method for the absorption product (20) according to claim 2, wherein an interval time between a start time of cutting the sensing layer (240) and a start time of cutting the leakage-proof bottom layer (220) is set according to a position of the sensing strip (241) relative to the receiving groove (221) in the longitudinal direction.

4. The manufacturing method for the absorption product (20) according to claim 2, wherein the sensing layer (240) is cut at a preset speed according to a conveying speed of the sensing layer (240).

5. The manufacturing method for the absorption product (20) according to claim 1, wherein a plurality of cutting marks (250) are spaced apart on the leakage-proof bottom layer (220) along the longitudinal direction.

6. The manufacturing method for the absorption product (20) according to any one of claims 1 to 5, wherein adhering the sensing strip (241) obtained by cutting to the side of the surface layer (210) facing the absorbent core (230) comprises:
adsorbing the sensing strip (241) obtained by cutting to a first cutting roller (271) configured to cut the sensing strip (241); and
controlling the first cutting roller (271) to rotate, so as to attach the adsorbed sensing strip (241) to the side of the surface layer (210) facing the absorbent core (230).

7. The manufacturing method for the absorption product (20) according to any one of claims 1 to 5, wherein controlling the cutting of the leakage-proof bottom layer (220) according to the in-position signal of the cutting mark (250) specifically comprises:
cutting the leakage-proof bottom layer (220), and sucking a waste material obtained by cutting into a hollow cavity (2721) of a second cutting roller (272) configured to cut the leakage-proof bottom layer (220), so that the waste material obtained by cutting is capable of being discharged through the hollow cavity (2721) of the second cutting roller (272).

8. The manufacturing method for the absorption product (20) according to claim 1, wherein a process of manufacturing the absorbent core (230) comprises:
supplying and conveying an absorbent core (230) layer in the longitudinal direction;
controlling a cutting of the absorbent core (230) layer according to the in-position signal of the cutting mark (250) to obtain the absorbent core (230) having the preset size; and
adhering the absorbent core (230) to a side of the leakage-proof bottom layer (220) facing the surface layer (210), so that an orthographic projection of the absorbent core (230) on the leakage-proof bottom layer (220) and the receiving groove (221) are staggered with each other.

9. A production system for manufacturing an absorption product (20), comprising a controller storing a computer program that, when executed by the controller, causes the controller to perform the steps of the manufacturing method according to any one of claims 1 to 8.

10. An absorption product (20), comprising:
a surface layer (210) arranged along a longitudinal direction;
a sensing strip (241) having a preset length along the longitudinal direction, the sensing strip (241) being adhered to a side of the surface layer (210);
a leakage-proof bottom layer (220) provided on a side of the surface layer (210) adhered to the sensing strip (241), wherein a receiving groove (221) configured to receive a data reader (260) is formed at a preset position of the leakage-proof bottom layer (220), and an orthographic projection of the sensing strip (241) on the leakage-proof bottom layer (220) intersects with the receiving groove (221); and
an absorbent core (230) provided between the surface layer (210) and the leakage-proof bottom layer (220);
wherein an end of the sensing strip (241) is capable of being connected to the data reader (260) in the receiving groove (221), and another end of the sensing strip (241) is capable of being in contact with the absorbent core (230).

## Patentansprüche

1. Herstellungsverfahren für ein Absorptionsprodukt (20), umfassend:
Zuführen und Fördern einer Oberflächenschicht (210) und einer auslaufsicheren Unterschicht (220) entlang einer Längsrichtung, wobei die Oberflächenschicht (210) und die auslaufsichere Unterschicht (220) einander gegenüberliegen und beabstandet sind und ein Absorptionskern (230) mit einer vorgegebenen Größe zwischen der Oberflächenschicht (210) und der auslaufsicheren Unterschicht (220) vorgesehen ist;
Zuführen und Fördern einer Sensorschicht (240), wobei die Sensorschicht (240) auf einer Seite der Oberflächenschicht (210) angeordnet ist;
Erfassen eines Positionssignals einer Schnittmarke (250) auf der auslaufsicheren Unterschicht (220) und Steuern eines Schneidens der Sensorschicht (240) nach dem Positionssignal der Schnittmarke (250), um einen Sensorstreifen (241) mit einer vorgegebenen Länge zu erhalten;
Anhaften des durch Schneiden erhaltenen Sensorstreifens (241) mit einer dem Absorptionskern (230) zugewandten Seite der Oberflächenschicht (210);
Steuern eines Schneidens der auslaufsicheren Unterschicht (220) nach dem Positionssignal der Schnittmarke (250), um an einer vorgegebenen Position der auslaufsicheren Unterschicht (220) eine Aufnahmenut (221) auszubilden, wobei die Aufnahmenut (221) zum Aufnehmen eines Datenlesegeräts (260) eingerichtet ist, und eine Orthogonalprojektion des Sensorstreifens (241) auf die auslaufsichere Unterschicht (220) sich mit der Aufnahmenut (221) überschneidet, und das Datenlesegerät (260) zum Erfassen von Durchgangsdaten des Sensorstreifens (241) eingerichtet ist; und
sequentielles Anhaften der Oberflächenschicht (210), des Absorptionskerns (230) und der auslaufsicheren Unterschicht (220), sodass ein Ende des Sensorstreifens (241) mit dem Datenlesegerät (260) in der Aufnahmenut (221) verbunden werden kann und ein anderes Ende des Sensorstreifens (241) in Kontakt mit dem Absorptionskern (230) sein kann.

2. Herstellungsverfahren für das Absorptionsprodukt (20) nach Anspruch 1, wobei das Steuern des Schneidens der Sensorschicht (240) nach dem Positionssignal der Schnittmarke (250), um den Sensorstreifen (241) mit der vorgegebenen Länge zu erhalten, insbesondere umfasst:
Festlegen einer Dauer eines kontinuierlichen Schneidens der Sensorschicht (240) nach dem Positionssignal der Schnittmarke (250);
wobei die Dauer des kontinuierlichen Schneidens der Sensorschicht (240) positiv mit der vorgegebenen Länge korreliert.

3. Herstellungsverfahren für das Absorptionsprodukt (20) nach Anspruch 2, wobei eine Intervallzeit zwischen einer Startzeit des Schneidens der Sensorschicht (240) und einer Startzeit des Schneidens der auslaufsicheren Unterschicht (220) nach einer Position des Sensorstreifens (241) relativ zu der Aufnahmenut (221) in der Längsrichtung festgelegt wird.

4. Herstellungsverfahren für das Absorptionsprodukt (20) nach Anspruch 2, wobei die Sensorschicht (240) mit einer vorgegebenen Geschwindigkeit nach einer Fördergeschwindigkeit der Sensorschicht (240) geschnitten wird.

5. Herstellungsverfahren für das Absorptionsprodukt (20) nach Anspruch 1, wobei eine Vielzahl von Schnittmarken (250) auf der auslaufsicheren Unterschicht (220) entlang der Längsrichtung beabstandet sind.

6. Herstellungsverfahren für das Absorptionsprodukt (20) nach einem der Ansprüche 1 bis 5, wobei das Anhaften des durch Schneiden erhaltenen Sensorstreifens (241) mit der dem Absorptionskern (230) zugewandten Seite der Oberflächenschicht (210) umfasst:
Ansaugen des durch Schneiden erhaltenen Sensorstreifens (241) an eine erste Schneidwalze (271), die zum Schneiden des Sensorstreifens (241) eingerichtet ist; und
Steuern der ersten Schneidwalze (271), sodass sie rotiert, um dadurch den angesaugten Sensorstreifen (241) an der dem Absorptionskern (230) zugewandten Seite der Oberflächenschicht (210) anzubringen.

7. Herstellungsverfahren für das Absorptionsprodukt (20) nach einem der Ansprüche 1 bis 5, wobei das Steuern des Schneidens der auslaufsicheren Unterschicht (220) nach dem Positionssignal der Schnittmarke (250) insbesondere umfasst:
Schneiden der auslaufsicheren Unterschicht (220) und Saugen von durch Schneiden erhaltenem Schnittabfall in einen Hohlraum (2721) einer zweiten Schneidwalze (272), die zum Schneiden der auslaufsicheren Unterschicht (220) eingerichtet ist, sodass der durch Schneiden erhaltene Schnittabfall durch den Hohlraum (2721) der zweiten Schneidwalze (272) abgeführt werden kann.

8. Herstellungsverfahren für das Absorptionsprodukt (20) nach Anspruch 1, wobei ein Prozess zum Herstellen des Absorptionskerns (230) umfasst:
Zuführen und Fördern einer Absorptionskern- (230) -schicht in der Längsrichtung;
Steuern eines Schneidens der Absorptionskern- (230) -schicht nach dem Positionssignal der Schnittmarke (250), um den Absorptionskern (230) mit der vorgegebenen Größe zu erhalten; und
Anhaften des Absorptionskerns (230) mit einer der Oberflächenschicht (210) zugewandten Seite der auslaufsicheren Unterschicht (220), sodass eine Orthogonalprojektion des Absorptionskerns (230) auf die auslaufsichere Unterschicht (220) und die Aufnahmenut (221) zueinander versetzt sind.

9. Herstellungssystem zum Herstellen eines Absorptionsprodukts (20), umfassend eine Steuereinheit, die ein Computerprogramm speichert, das, wenn es durch die Steuereinheit ausgeführt wird, die Steuereinheit veranlasst, die Schritte des Herstellungsverfahrens nach einem der Ansprüche 1 bis 8 auszuführen.

10. Absorptionsprodukt (20), umfassend:
eine Oberflächenschicht (210), die entlang einer Längsrichtung angeordnet ist;
einen Sensorstreifen (241) mit einer vorgegebenen Länge entlang der Längsrichtung, wobei der Sensorstreifen (241) an einer Seite der Oberflächenschicht (210) anhaftend ist;
eine auslaufsichere Unterschicht (220), die auf einer an der Sensorstreifen (241) anhaftenden Seite der Oberflächenschicht (210) vorgesehen ist, wobei an einer vorgegebenen Position der auslaufsicheren Unterschicht (220) eine Aufnahmenut (221) ausgebildet ist, die zum Aufnehmen eines Datenlesegeräts (260) eingerichtet ist, und eine Orthogonalprojektion des Sensorstreifens (241) auf die auslaufsichere Unterschicht (220) sich mit der Aufnahmenut (221) überschneidet; und
einen Absorptionskern (230), der zwischen der Oberflächenschicht (210) und der auslaufsicheren Unterschicht (220) vorgesehen ist;
wobei ein Ende des Sensorstreifens (241) mit dem Datenlesegerät (260) in der Aufnahmenut (221) verbunden werden kann und ein anderes Ende des Sensorstreifens (241) in Kontakt mit dem Absorptionskern (230) sein kann.

## Revendications

1. Procédé de fabrication d'un produit d'absorption (20), comprenant :
l'apport et l'acheminement d'une couche de surface (210) et d'une couche inférieure anti-fuite (220) le long d'une direction longitudinale, dans lequel la couche de surface (210) et la couche inférieure anti-fuite (220) sont en regard l'une de l'autre et espacées l'une de l'autre, et une âme absorbante (230) présentant une taille prédéfinie est disposée entre la couche de surface (210) et la couche inférieure anti-fuite (220) ;
l'apport et l'acheminement d'une couche de détection (240), la couche de détection (240) étant située sur un côté de la couche de surface (210) ;
l'acquisition d'un signal de positionnement d'un repère de découpe (250) sur la couche inférieure anti-fuite (220), et la commande d'une découpe de la couche de détection (240) en fonction du signal de positionnement du repère de découpe (250) afin d'obtenir une bande de détection (241) présentant une longueur prédéfinie ;
le collage de la bande de détection (241), obtenue par découpe, à un côté de la couche de surface (210) faisant face à l'âme absorbante (230) ;
la commande d'une découpe de la couche inférieure anti-fuite (220) en fonction du signal de positionnement du repère de coupe (250), de manière à former une rainure de réception (221) à une position prédéfinie de la couche inférieure anti-fuite (220), dans lequel la rainure de réception (221) est configurée pour recevoir un lecteur de données (260), et une projection orthogonale de la bande de détection (241) sur la couche inférieure anti-fuite (220) croise la rainure de réception (221), et le lecteur de données (260) est configuré pour acquérir une donnée de conduction de la bande de détection (241) ; et
le collage successif de la couche de surface (210), l'âme absorbante (230) et de la couche inférieure anti-fuite (220), de telle sorte qu'une extrémité de la bande de détection (241) est apte à être reliée au lecteur de données (260) dans la rainure de réception (221), et qu'une autre extrémité de la bande de détection (241) est apte à être en contact avec l'âme absorbante (230).

2. Procédé de fabrication du produit d'absorption (20) selon la revendication 1, dans lequel la commande de la découpe de la couche de détection (240) en fonction du signal de positionnement du repère de découpe (250) afin d'obtenir la bande de détection (241) présentant la longueur prédéfinie comprend spécifiquement :
le réglage d'une durée de découpe continue de la couche de détection (240) en fonction du signal de positionnement du repère de découpe (250) ;
dans lequel la durée de la découpe continue de la couche de détection (240) est corrélée positivement avec la longueur prédéfinie.

3. Procédé de fabrication du produit d'absorption (20) selon la revendication 2, dans lequel un intervalle de temps entre un temps de début de découpe de la couche de détection (240) et un temps de début de découpe de la couche inférieure anti-fuite (220) est réglé en fonction d'une position de la bande de détection (241) par rapport à la rainure de réception (221) dans la direction longitudinale.

4. Procédé de fabrication du produit d'absorption (20) selon la revendication 2, dans lequel la couche de détection (240) est découpée à une vitesse prédéfinie en fonction d'une vitesse d'acheminement de la couche de détection (240).

5. Procédé de fabrication du produit d'absorption (20) selon la revendication 1, dans lequel une pluralité de repères de découpe (250) sont espacés sur la couche inférieure anti-fuite (220) le long de la direction longitudinale.

6. Procédé de fabrication du produit d'absorption (20) selon l'une quelconque des revendications 1 à 5, dans lequel le collage de la bande de détection (241), obtenue par découpe, au côté de la couche de surface (210) faisant face à l'âme absorbante (230) comprend :
l'adsorption de la bande de détection (241), obtenue par découpe, sur un premier rouleau de découpe (271) configuré pour découper la bande de détection (241) ; et
la commande de la rotation du premier rouleau de découpe (271), de manière à fixer la bande de détection adsorbée (241) au côté de la couche de surface (210) faisant face à l'âme absorbante (230).

7. Procédé de fabrication du produit d'absorption (20) selon l'une quelconque des revendications 1 à 5, dans lequel la commande de la découpe de la couche inférieure anti-fuite (220) en fonction du signal de positionnement du repère de découpe (250) comprend spécifiquement :
la découpe de la couche inférieure anti-fuite (220), et l'aspiration d'un déchet de découpe dans une cavité creuse (2721) d'un deuxième rouleau de découpe (272) configuré pour découper la couche inférieure anti-fuite (220), de telle sorte que le déchet de découpe est apte à être évacué par la cavité creuse (2721) du deuxième rouleau de découpe (272).

8. Procédé de fabrication du produit d'absorption (20) selon la revendication 1, dans lequel un processus de fabrication de l'âme absorbante (230) comprend :
l'apport et l'acheminement d'une couche d'âme absorbante (230) dans la direction longitudinale ;
la commande d'une découpe de la couche d'âme absorbante (230) en fonction du signal de positionnement du repère de découpe (250) afin d'obtenir l'âme absorbante (230) présentant la taille prédéfinie ; et
le collage de l'âme absorbante (230) à un côté de la couche inférieure anti-fuite (220) faisant face à la couche de surface (210), de telle sorte qu'une projection orthogonale de l'âme absorbante (230) sur la couche inférieure anti-fuite (220) et la rainure de réception (221) sont décalées l'une par rapport à l'autre.

9. Système de production pour la fabrication d'un produit d'absorption (20), comprenant un dispositif de commande stockant un programme informatique qui, lorsqu'il est exécuté par le dispositif de commande, amène le dispositif de commande à réaliser les étapes du procédé de fabrication selon l'une quelconque des revendications 1 à 8.

10. Produit d'absorption (20), comprenant :
une couche de surface (210) disposée le long d'une direction longitudinale ;
une bande de détection (241) présentant une longueur prédéfinie le long de la direction longitudinale, la bande de détection (241) étant collée à un côté de la couche de surface (210) ;
une couche inférieure anti-fuite (220) disposée sur un côté de la couche de surface (210) collé à la bande de détection (241), dans lequel une rainure de réception (221) configurée pour recevoir un lecteur de données (260) est formée à une position prédéfinie de la couche inférieure anti-fuite (220), et une projection orthogonale de la bande de détection (241) sur la couche inférieure anti-fuite (220) croise la rainure de réception (221) ; et
une âme absorbante (230) disposée entre la couche de surface (210) et la couche inférieure anti-fuite (220) ;
dans lequel une extrémité de la bande de détection (241) est apte à être reliée au lecteur de données (260) dans la rainure de réception (221), et une autre extrémité de la bande de détection (241) est apte à être en contact avec l'âme absorbante (230).
